# EUROPEAN PATENT APPLICATION

(11) **EP 4 610 363 A1**
(43) Date of publication of application: **03.09.2025**
(21) Application number: 24000149.5
(22) Date of filing: 23.12.2024
(51) Int. Cl.: C12P 7/6436, C12N 9/18, C12P 17/04

(54) **METHOD OF PRODUCING FURFURYL ESTERS**

(30) Priority: 29.02.2024 PL 44789924
(71) Applicant: Politechnika Slaska, 44-100 Gliwice (PL)
(72) Inventor: Wolny, Anna, 41-506 CHORZÓW (PL); Chrobok, Anna, 42-674 ZBROSLAWICE (PL)

(57) **Abstract**

Method for producing furfuryl esters involving conducting the esterification reaction of furfuryl alcohol and a carboxylic acid in a batch process or continuously, in the presence of an organic solvent. The biocatalyst is an *Aspergillus oryzae* lipase (AOL), either native or physically immobilized on MgO·SiO₂-C₈-AOL or MgO·SiO₂-C₁₆-AOL supports. The process is carried out at temperatures ranging from 18°C to 40°C and the remaining post-reaction mixture is subjected to vacuum distillation.

## Description

The subject of the invention is a method for obtaining furfuryl esters, which are used in the production of biofuels, polymers (bio-lubricants, plasticizers), food flavorings, and cosmetic products. Furfuryl alcohol esters are high-value products, and the substrate used-furfuryl alcohol-can be obtained through the processing of lignocellulosic biomass.

The instability of furfuryl alcohol in an acidic environment makes it impossible to obtain furfuryl esters using traditional acidic catalysts typically employed in esterification. Furfuryl alcohol undergoes polymerization in the presence of mineral acids (T. T. Mokoena, W. A. A. Ddamba, B. M. Keikotlhaile, S. Afr. J. Chem 1999, 52; E. M. Wewerka, J. Appl. Polym. Sci. 1968, 12, 1671) or strong organic acids (R. González, R. Martinez, P. Ortiz, Die Makromol. Chemie 1992, 193, 1), forming thermally stable polymers (H. E. Hoydonckx, D. E. De Vos, S. A. Chavan, P. A. Jacobs, Top. Catal. 2004, 27, 83) or difurfuryl ethers (R. S. Malkar, H. Daly, C. Hardacre, G. D. Yadav, React. Chem. Eng. 2019, 4, 1790-1802), which presents a significant obstacle to the synthesis of furfuryl esters.

To obtain furfuryl esters through esterification, furfuryl alcohol is used along with a carboxylic acid, carboxylic acid halide, ester, or acid anhydride in the presence of metallic, acidic, or enzymatic catalysts. Known methods for synthesizing furfuryl esters from furfuryl alcohol and various carboxylic acids are as follows.

A literature example of furfuryl ester synthesis involves the esterification reaction of furfuryl alcohol using acetic acid in a molar ratio of 1:5, with a Lewis acidic catalyst based on zirconium oxide and yttrium oxide, without a solvent, at a high temperature of 110°C and a reaction time of 7 hours. A yield of 88% of the ester was achieved (P. Kumar, R. K. Pandey, M. S. Bodas, S. P. Dagade, M. K. Dongare, A. V. Ramaswamy, J. Mol. Catal. A Chem. 2002, 181, 207).

A non-patent literature method for obtaining furfuryl esters involves the reaction of furfuryl alcohol and acetic acid in a molar ratio of 1:6 in the presence of a heteropolyacid doped with iron, zirconium, aluminum, or cobalt, encapsulated in a ZIF-8 type zeolite, at a high temperature of 90°C for 6 hours. The selectivity of ester formation reached 91%, with a furfuryl alcohol conversion rate of 86% (R. S. Malkar, H. Daly, C. Hardacre, G. D. Yadav, React. Chem. Eng. 2019, 4, 1790-1802).

From the European patent description EP1013632A1, a method is known for obtaining furfuryl esters through the esterification of furfuryl alcohol and acetic acid in molar ratios of 1:3-11 in the presence of a catalyst based on montmorillonites/metallic ion-exchanged clays containing cations of iron, copper, zinc, aluminum, cesium, or zirconium. The reaction is conducted in a hydrocarbon organic solvent (e.g., dichloromethane, toluene) or without a solvent, at a temperature of 30-140°C, for a duration ranging from 0.02 to 3 hours. The furfuryl ester was isolated with a yield of 65%.

An alternative method for obtaining furfuryl esters, described in non-patent literature, involves the esterification of furfuryl alcohol and oleic acid in a molar ratio of 1:1 using the biocatalyst Novozym 435 at a temperature of 60°C for 6 hours. The conversion rate of oleic acid reached 99% (A. Sengupta, T. Dey, M. Ghosh, J. Ghosh, S. Ghosh, J. Inst. Eng. Ser. E. 2012, 93, 31).

From the non-patent literature by Y. Satyawali, V. Akemeier, W. Dejonghe, H. De Wever, and W. Van Hecke, Waste and Biomass Valorization 2019, 10, 311, the use of the same biocatalyst, Novozym 435, is known for the esterification of furfuryl alcohol and octanoic acid in a molar ratio of 1:1, in the presence of a drying agent, at a temperature of 55°C for 24 hours. The conversion rate of furfuryl alcohol was 73%.

Furthermore, from the non-patent literature by S. Mukherjee, M. Ghosh, Carbohydr. Polym., 2017, 157, 1076-1084, a method is known for synthesizing furfuryl esters through the esterification of furfuryl alcohol and castor oil in a molar ratio of 3:1, using Novozym 435 as the catalyst, at a temperature of 60°C for 5 hours. A yield of 89% for the furfuryl ester was achieved.

From the non-patent literature, a method is known for obtaining furfuryl esters through the reaction between furfuryl alcohol and furan-2-carboxylic acid in a molar ratio of 2:2.6, in the presence of para-toluenesulfonate 4-(4,6-dimethoxy-1,3,5-triazine-2-yl)-4-methylmorpholine in dichloromethane (3 mL) in a microwave reactor at a temperature of 90°C for 30 minutes, with a synthesis yield of 49%. In the same publication, the reaction between furfuryl alcohol and furan-2-carboxylic acid in a molar ratio of 2:6 is also described, using 1-ethyl-3-(3-dimethylaminopropyl)carbodiimide in dichloromethane (3 mL) in a microwave reactor at 90°C for 30 minutes, achieving a synthesis yield of 71% (L. Janczewski, D. Zieliński, B. Kolesińska, ChemistryOpen, 2021, 19, 265-280).

The technical problem that needs to be solved is the development of a new, highly selective and efficient method for obtaining furfuryl esters.

The method for obtaining furfuryl esters involves conducting the esterification reaction in a batch process, using 1 mmol of furfuryl alcohol, to which 1 mmol to 10 mmol of carboxylic acid is added. The resulting mixture is dissolved in organic solvents in amounts ranging from 0.5 mL to 3 mL, then 10 mg to 300 mg of a biocatalyst in the form of AOL lipase, either native or physically immobilized on a MgO·SiO₂-C₈-AOL or MgO·SiO₂-C₁₆-AOL carrier, is added. Preferably, 150 mg of lipase is used per 1 mmol of furfuryl alcohol. The reaction is carried out in a shaker at temperatures ranging from 18°C to 40°C, for a duration of 15 minutes to 300 minutes. Afterward, the biocatalyst is filtered out, and the post-reaction mixture is separated by vacuum distillation.

The method for obtaining furfuryl esters involves conducting the esterification reaction continuously, with a reagent flow rate of 20 µL to 200 µL per minute. A solution of furfuryl alcohol in an organic solvent with a concentration of 2.0 mol/dm³ is supplied in a 1:3 ratio along with the carboxylic acid. The mixture is then passed through a reactor filled with a biocatalyst in the form of AOL lipase, physically immobilized on a MgO·SiO₂-C₈-AOL or MgO·SiO₂-C₁₆-AOL carrier. The process is carried out at temperatures ranging from 18°C to 40°C for a duration of 4 minutes to 37 minutes. Afterward, the organic solvent is distilled from the post-reaction mixture and returned to the esterification process, while the remaining post-reaction mixture is subjected to vacuum distillation.

Preferably, in the method for obtaining furfuryl esters according to the invention, the biocatalyst used is AOL lipase physically immobilized on a MgO·SiO₂-C₈ or MgO·SiO₂-C₁₆ carrier, obtained by modifying the MgO·SiO₂ carrier with alkyl groups C₈ or C₁₆ in a ratio of 0.5 mmol to 5 mmol of alkyl groups per 1 g of carrier. This modification is carried out using a mixture of isopropanol/water in a 4:1 (v/v) ratio, with a volume of 4 mL to 20 mL, at a temperature ranging from 85°C to 100°C while mixing for a duration of 4 to 24 hours. The material is then filtered, washed with isopropanol, and dried in a rotary evaporator for 1 to 6 hours. The modified MgO·SiO₂-C₈ or MgO·SiO₂-C₁₆ carrier and AOL lipase are mixed in a mass ratio of 1:3-8, demineralized water is added, and the mixture is stirred at a temperature from 17°C to 30°C for 1 to 24 hours. The material is then filtered, washed with demineralized water, and dried.

Preferably, in the method for obtaining furfuryl esters according to the invention, the carboxylic acids used are octanoic acid, nonanoic acid, decanoic acid, lauric acid, and oleic acid.

Preferably, in the method for obtaining furfuryl esters according to the invention, the organic solvents used are cyclohexane, hexane, iso-octane, heptane, toluene, and dichloromethane.

Preferably, in the method for obtaining furfuryl esters according to the invention, the biocatalytic bed consists of 300 mg per 2.0 mol/dm³ solution of furfuryl alcohol in an organic solvent.

The subject of the invention is presented in the following examples of implementation.

### Example 1

### The method for synthesizing lipase from Aspergillus oryzae physically immobilized on a carrier

In a 25 mL round-bottom flask equipped with a reflux condenser, 300 mg of the MgO·SiO₂ carrier and 0.3 mmol of triethoxy-octylsilane are introduced. Then, 4 mL of an isopropanol/water mixture (4:1, v/v) is added, and the mixture is stirred at 85°C for 6 hours. Afterward, the material is filtered, washed with 20 mL of isopropanol, and dried in a rotary evaporator for 2 hours. The amount of octyl groups in the MgO·SiO₂-C₈ was 8.03%.
Next, 300 mg of MgO·SiO₂-C₈ and 6 times the mass of AOL lipase, relative to the carrier, are added to a 25 mL round-bottom flask. Then, 4 mL of demineralized water is added, and the contents are mixed on a shaker at 20°C for 3 hours. The material is then filtered, washed with demineralized water until excess lipase is removed, and dried under vacuum on a Schlenk line (at 20°C for 24 hours). The amount of lipase immobilized in the MgO·SiO₂-C₈-AOL biocatalyst was 4.24%.

### Example 2

### The method for synthesizing lipase from Aspergillus oryzae physically immobilized on a carrier

In a 25 mL round-bottom flask equipped with a reflux condenser, 300 mg of MgO·SiO₂ carrier and 1.5 mmol of triethoxy-octylsilane are introduced. Then, 4 mL of an isopropanol/water mixture (4:1, v/v) is added, and the mixture is stirred at 85°C for 4 hours. Afterward, the material is filtered, washed with 20 mL of isopropanol, and dried in a rotary evaporator for 1 hour. The amount of octyl groups in the MgO·SiO₂-C₈ was 5.96%.
Next, 300 mg of MgO·SiO₂-C₈ and 3 times the mass of AOL lipase relative to the carrier are added to a 25 mL round-bottom flask. Then, 4 mL of demineralized water is added, and the contents are mixed on a shaker at 17°C for 1 hour. The material is then filtered, washed with demineralized water until excess lipase is removed, and dried under vacuum on a Schlenk line (at 20°C for 24 hours). The amount of lipase immobilized in the MgO·SiO₂-C₈-AOL biocatalyst was 3.41%.

### Example 3

### The method for synthesizing lipase from Aspergillus oryzae physically immobilized on a carrier

In a 25 mL round-bottom flask equipped with a reflux condenser, 300 mg of MgO·SiO₂ carrier and 0.15 mmol of triethoxyhexadecylsilane are introduced. Then, 20 mL of an isopropanol/water mixture (4:1, v/v) is added, and the mixture is stirred at 100°C for 24 hours. Afterward, the material is filtered, washed with 20 mL of isopropanol, and dried in a rotary evaporator for 6 hours. The amount of octyl groups in the MgO·SiO₂-C₁₆ was 5.87%.
Next, 300 mg of MgO·SiO₂-C₁₆ and 8 times the mass of AOL lipase relative to the carrier are added to a 25 mL round-bottom flask. Then, 4 mL of demineralized water is added, and the contents are mixed on a shaker at 30°C for 24 hours. The material is then filtered, washed with demineralized water until excess lipase is removed, and dried under vacuum on a Schlenk line (at 20°C for 24 hours). The amount of lipase immobilized in the MgO·SiO₂-C₁₆-AOL biocatalyst was 1.57%.

### Example 4

### Method of producingfurfuryl esters

In a 10 mL round-bottom flask, furfuryl alcohol (1 mmol), carboxylic acid (3 mmol), cyclohexane (0.5 mL), and the biocatalyst MgO·SiO₂-C₈-AOL are introduced. The contents of the flask are shaken in a shaker (250 rpm) at 25°C for 45 to 90 minutes. The results of the conversion obtained within the given time for the respective carboxylic acid are presented in Table 1.

**Table 1. The conversion of furfuryl alcohol to the respective furfuryl ester.**

| **Carboxylic acid** | **Time** | **Conversion* [%]** | **Selectivity [%]** |
|---|---|---|---|
| Octanoic acid | 45 min | 87 | 100 |
| Nonanoic acid | 60 min | 87 | 100 |
| Decanoic acid | 60 min | 89 | 100 |
| Lauric acid | 60 min | 86 | 100 |
| Oleic acid | 90 min | 90 | 100 |

| | | | |
|---|---|---|---|
| *The conversion is calculated with respect to furfuryl alcohol *Reaction conditions:* 1 mmol furfuryl alcohol, 3 mmol carboxylic acid, 0.5 mL cyclohexane, 25 °C, 250 rpm, 150 mg MgO·SiO₂-C₈-AOL. | | | |

### Example 5

### Method of producing furfuryl octanoate

In a 10 mL round-bottom flask, furfuryl alcohol (1 mmol), octanoic acid (1 mmol), hexane (0.5 mL), and the native form of AOL biocatalyst (10 mg) are introduced. The contents of the flask are shaken in a shaker (250 rpm) at 18°C for 15 minutes. The conversion of furfuryl alcohol to furfuryl octanoate was 10%, with a selectivity of 100%.

### Example 6

### Method of producing furfuryl octanoate

In a 10 mL round-bottom flask, furfuryl alcohol (1 mmol), octanoic acid (10 mmol), toluene (3 mL), and the biocatalyst MgO·SiO₂-C₁₆-AOL (300 mg) are introduced. The contents of the flask are shaken in a shaker (250 rpm) at 40°C for 300 minutes. The conversion of furfuryl alcohol to furfuryl octanoate was 88%, with a selectivity of 100%.

### Example 7

### Method of producing furfuryl octanoate

In a 10 mL round-bottom flask, furfuryl alcohol (1 mmol), carboxylic acid (3 mmol), iso-octane (0.5 mL), and the biocatalyst MgO·SiO₂-C₈-AOL are introduced. The contents of the flask are shaken in a shaker (250 rpm) at 25°C for 180 minutes. The conversion of furfuryl alcohol to furfuryl octanoate was 38%, with a selectivity of 100%.

### Example 8

### Method of producing furfuryl octanoate

In a 10 mL round-bottom flask, furfuryl alcohol (1 mmol), carboxylic acid (3 mmol), dichloromethane (0.5 mL), and the biocatalyst MgO·SiO₂-C₈-AOL are introduced. The contents of the flask are shaken in a shaker (250 rpm) at 25°C for 180 minutes. The conversion of furfuryl alcohol to furfuryl octanoate was 27%, with a selectivity of 100%.

### Example 9

### Method of producing furfuryl octanoate

In a column reactor with an inner diameter of 10 mm and a height of 100 mm, filled with 300 mg of the biocatalyst MgO·SiO₂-C₈-AOL (with a bed volume of 0.735 mL), a continuous flow of 70 µL per minute is introduced. The flow consists of a solution of furfuryl alcohol (14.727 g) in cyclohexane (75 mL) and octanoic acid (94.917 g). The process is conducted under a pressure of 2 bar and at a temperature of 25 °C. The product is collected with a yield of 97%. The residence time of the substrates in the reactor is 10.5 minutes.

### Example 10

### Method of producingfurfuryl octanoate

In a column reactor with an inner diameter of 10 mm and a height of 100 mm, filled with 300 mg of the biocatalyst MgO·SiO₂-C₁₆-AOL (with a bed volume of 0.735 mL), a continuous flow of 20 µL per minute is introduced. The flow consists of a solution of furfuryl alcohol (14.727 g) in toluene (75 mL) and octanoic acid (64.890 g). The process is conducted under a pressure of 2 bar and at a temperature of 18 °C. The product is collected with a yield of 97%. The contact time of the reagents with the biocatalyst is 37 minutes.

### Example 11

### Method of producingfurfuryl octanoate

In a column reactor with an inner diameter of 10 mm and a height of 100 mm, filled with 300 mg of the biocatalyst MgO·SiO₂-C₈-AOL (with a bed volume of 0.735 mL), a continuous flow of 200 µL per minute is introduced. The flow consists of a solution of furfuryl alcohol (14.727 g) in hexane (75 mL) and octanoic acid (94.917 g). The process is conducted under a pressure of 2 bar and at a temperature of 40 °C. The product is collected with a yield of 79%. The contact time of the reagents with the biocatalyst is 4 minutes. The process for the synthesis of furfuryl esters, both in batch and continuous modes, can be scaled, considering the parameters defined in the patent claims.

The advantage of the solution according to the invention lies in the new possibilities for the synthesis of furfuryl esters, which allow for the simultaneous achievement of high conversion and selectivity values in a short time. The method for obtaining furfuryl esters eliminates the potential polymerization of furfuryl alcohol. The proposed solution in the continuous system further increases the efficiency of the furfuryl ester synthesis process. The use of commercially available lipase from *Aspergillus oryzae* in its native form, and its immobilization on silica-based supports, additionally facilitates the separation of the solid biocatalyst from the post-reaction mixture, allowing for its return to the next reaction cycle. This enables the production of an unpolluted product, free from biocatalyst residues, and offers the potential for industrial-scale implementation.

## Claims

1. A method for producing furfuryl esters, **characterized in that** the esterification reaction is carried out periodically, in the presence of 1 mmol of furfuryl alcohol, to which from 1 mmol to 10 mmol of a carboxylic acid is added. The obtained mixture is dissolved in organic solvents in an amount from 0.5 ml to 3 ml, then from 10 mg to 300 mg of a biocatalyst in the form of AOL lipase, either native or physically immobilized on a MgO·SiO₂-C₈-AOL or MgO·SiO₂-C₁₆-AOL carrier, preferably 150 mg of lipase per 1 mmol of furfuryl alcohol, is added. The reaction is carried out in a shaker at temperatures ranging from 18°C to 40°C for 15 to 300 minutes, after which the biocatalyst is filtered out, and the post-reaction mixture is separated by vacuum distillation.

2. A method for producing furfuryl esters, **characterized in that** the esterification reaction is carried out continuously, with a reagent flow rate of 20 µl to 200 µl per minute, while simultaneously adding a solution of furfuryl alcohol in an organic solvent with a concentration of 2.0 mol/dm³ in a 1:3 ratio, along with a carboxylic acid. The mixture is then fed into a reactor filled with a biocatalyst in the form of AOL lipase physically immobilized on a MgO·SiO₂-C₈-AOL or MgO·SiO₂-C₁₆-AOL carrier. The process is conducted at a temperature from 18°C to 40°C, for a duration of 4 minutes to 37 minutes. Afterward, the organic solvent is distilled off from the post-reaction mixture and returned to the esterification process, while the remaining post-reaction mixture undergoes vacuum distillation.

3. A method for producing esters according to claims 1 and 2, **characterized in that** the biocatalyst used is AOL lipase physically immobilized on a MgO·SiO₂-C₈ or MgO·SiO₂-C₁₆ carrier obtained by modifying the MgO·SiO₂ carrier with C₈, C₁₆ alkyl groups in the range from 0.5 mmol to 5 mmol of alkyl groups per 1 g of the carrier using 4 ml to 20 ml of an isopropanol/water mixture in a 4:1 ratio (v/v). The process is conducted at a temperature from 85°C to 100°C, with stirring for 4 to 24 hours, after which the material is filtered, washed with isopropanol, and dried in a rotary evaporator for 1 to 6 hours. The modified MgO·SiO₂-C₈ or MgO·SiO₂-C₁₆ carrier is then mixed with AOL lipase in a mass ratio of 1:3-8, demineralized water is added, and the mixture is stirred at a temperature from 17°C to 30°C for 1 to 24 hours. The material is then filtered, washed with demineralized water, and dried.

4. A method for producing esters according to claims 1 and 2, **characterized in that** the carboxylic acids used are octanoic acid, nonanoic acid, decanoic acid, lauric acid, or oleic acid.

5. A method for producing esters according to claims 1 and 2, **characterized in that** the organic solvents used are cyclohexane, hexane, isooctane, heptane, toluene, or dichloromethane.

6. A method for producing esters according to claim 2 is **characterized in that** the biocatalytic bed consists of 300 mg per 2.0 mol/dm³ solution of furfuryl alcohol in an organic solvent.
